# EUROPÄISCHE PATENTANMELDUNG

(11) **EP 3 960 148 A1**
(43) Veröffentlichungstag der Anmeldung: **02.03.2022**
(21) Anmeldenummer: 20193325.6
(22) Anmeldetag: 28.08.2020
(51) Int. Cl.: A61K 8/25, A61K 8/26, A61K 8/28, A61K 8/29, A61K 8/34, A61K 8/46, A61Q 11/00

(54) **DENTALE PROPHYLAXEPASTE**

(71) Anmelder: Ivoclar Vivadent AG, 9494 Schaan (LI)
(72) Erfinder: Lendenmann, Urs, 9472 Grabs (CH); GEITER, Ulrich, FL-9393 Mauren (LI); BECKER, Philipp, 9475 Sevelen (CH); OELMANN, Marc, 9470 Buchs (CH); SCHOPPE, Janina, 6800 Feldkirch (AT)
(74) Vertreter: Uexküll & Stolberg

(57) **Zusammenfassung**

Dentale Polier- und Reinigungspaste für die professionelle Zahnreinigung, die 1 bis 30 Gew.-% Wasser, 25 bis 70 Gew.-% mindestens eines Feuchthaltemittels, 25 bis 60 Gew.-% mindestens eines Putzkörpers und 0,05 bis 2 Gew.-% mindestens eines Tensids enthält, jeweils bezogen auf die Gesamtmasse der Polier- und Reinigungspaste.

## Beschreibung

Die Erfindung betrifft Zusammensetzungen, die sich besonders als dentale Polier- und Reinigungspasten eignen. Die Pasten weisen eine geschmeidige Konsistenz auf und zeichnen sich dadurch aus, dass sie an der Luft nicht austrocken oder zerfließen.

### Stand der Technik

Bakterielle Plaque gefährdet Zahnhartsubstanz und Zahnhalteapparat. Werden keine Schutzmaßnahmen ergriffen, kann es zu einer Demineralisation der Zähne kommen, die schlussendlich in Karies mündet. Eine supragingivale Plaqueansammlung kann auch zu parodontalen Erkrankungen führen, zum Beispiel zu Gingivitis, Parodontitis oder auch zu Mukositis oder Periimplantitis. Daher ist es nötig, Oberflächen innerhalb des Mundes so plaquefrei wie möglich zu halten. Das gilt sowohl für natürliche als auch für restaurierte oder durch Implantate ersetzte Zähne.

Neben der häuslichen Mundhygiene mit Zahnbürste und Zahnpaste leisten die professionelle Zahnreinigung und -politur einen wichtigen Beitrag zur Reduktion des Biofilms. Die Professionelle Zahnreinigung umfasst die Entfernung supragingivaler Zahnbeläge inkl. Zahnstein, das Beseitigen von Verfärbungen sowie das Glätten und Polieren rauer Oberflächen.

Die hierzu verwendeten Polier- und Reinigungspasten ähneln in ihrer Zusammensetzung den üblichen Zahnpasten. Sie eignen sich jedoch nicht zur täglichen häuslichen Zahnpflege, weil sie für die speziellen Anforderungen der professionellen Zahnreinigung konzipiert sind. Prophylaxepasten sollen eine maximale Reinigungswirkung bieten, um Verfärbungen und Beläge wirksam zu entfernen. Außerdem sollen die Oberflächen nach dem Polieren möglichst glatt sein.

Prophylaxepasten enthalten Putzkörper, wie zum Beispiel amorphes Siliciumdioxid, Calciumcarbonat, Calciumphosphate, Hydroxyapatitpulver, Bimsmehl, die in einer flüssigen Matrix suspendiert sind. Die Matrix besteht meist aus Wasser, Glycerin, Polyethylenglycol (PEG), Propylenglycol und ähnlichen oral akzeptablen Lösungsmitteln.

Natürlicher Zahnschmelz ist ein sehr hartes Gewebe und daher hinsichtlich der Wahl der Putzkörper wenig kritisch. Demgegenüber weisen die zur Restauration geschädigter Zähne verwendeten Materialien, wie z.B. Kunststofffüllungsmaterialien und dentale Metalllegierungen, eine deutlich geringere Härte auf, und es besteht daher die Gefahr, dass diese bei der Wahl ungeeigneter Putzkörper zerkratzt werden. Kratzer begünstigen die Ansiedelung von Plaque und sind damit nicht nur ein kosmetisches Problem. Andere Putzkörper wie z.B. amorphe Kieselsäuren sind in dieser Hinsicht unbedenklich, haben jedoch eine deutlich geringere Reinigungswirkung und erfordern so einen erheblich höheren zeitlichen Putzaufwand, um ein vergleichbares Reinigungsergebnis zu erzielen. Stärkere Verfärbungen lassen sich damit nicht von der Zahnoberfläche entfernen.

Zahn- und Prophylaxepasten müssen eine genügend hohe Viskosität aufweisen, damit die Putzkörper nicht sedimentieren und die Pasten auf Zahnbürsten oder dentalen Reinigungs-und Polierinstrumenten dosiert werden können, ohne wegzufließen. Zur Einstellung der Viskosität werden gewöhnlich Verdickungsmittel eingesetzt. Hierbei handelt es sich meist um synthetische oder natürliche Polymere.

Dentale Prophylaxepasten für den professionellen Einsatz werden aus hygienischen Gründen in der Regel als Einzelverpackungen verwendet, die die für eine einmalige Anwendung erforderliche Produktmenge, meist 1,5 bis 2 g, enthalten. Nachteilig ist, dass die Materialen wegen der geringen Produktmenge nach dem Öffnen der Verpackung schnell austrocknen und dann nur noch schwer einsetzbar sind. Außerdem werden aus Kostengründen häufig Verpackungsmaterialen eingesetzt, die nicht vollständig dampfdicht sind, so dass selbst die verpackten Materialien nur begrenzt haltbar sind. Zur Verhinderung einer vorzeitigen Austrocknung enthalten die Pasten in der Regel Feuchthaltemittel. Viele Feuchthaltemittel sind jedoch hygroskopisch, so dass die Produkte in feuchter Umgebung Wasser aufnehmen. Hierdurch werden die Materialen verdünnt und zerfließen. Es kann auch vorkommen, dass sich auf der Oberfläche des Materials ein flüssiger Film bildet, was als Ausbluten bezeichnet wird, der die Verarbeitungseigenschaften der Paste ebenfalls beeinträchtigt.

Die WO 2009/120854 A2 offenbart Mundpflegeprodukte, die Gelnetzwerke enthalten. Unter Gelnetzwerken werden lamellare oder vesikuläre, feste Kristallphasen verstanden, die ein Fettamphiphil, Tenside und Lösungsmittel enthalten. Zur Herstellung der Gelnetzwerke wird das Fettamphiphil zusammen mit einem dispergierenden Tensid in dem Lösungsmittel dispergiert. Anschließend wird die erhaltende Dispersion mit einem quellenden Tensid gemischt. Die Zugabe des quellenden Tensids soll die Ausbildung eines Gelnetzwerks bewirken, das eine verdickende Wirkung haben soll.

Die WO 2010/068424 A2 offenbart Mundpflegemittel, die neben einem Gelnetzwerk Quarzglas mit einer mittleren Partikelgröße von 3 bis 15 µm als Putzkörper enthalten.

### Kurzbeschreibung der Erfindung

Der Erfindung liegt die Aufgabe zugrunde, eine dentale Prophylaxepaste, d.h. eine dentale Polier- und Reinigungspaste, zur Verfügung zu stellen, die nicht die Nachteile bekannter Produkte aufweist, die insbesondere nicht vorzeitig austrocknet und die eine für die geplante Anwendung optimierte Konsistenz hat. Die Pasten sollen eine effiziente und schnelle Reinigung natürlicher und künstlicher Zahnoberflächen ermöglichen, ohne die Oberflächen zu zerkratzen.

Erfindungsgemäß wird diese Aufgabe durch Pasten gelöst, die
- 1 bis 30 Gew.-%, bevorzugt 5 bis 25 Gew.-% und besonders bevorzugt 10 bis 20 Gew-% Wasser,
- 25 bis 70 Gew.-%, bevorzugt 30 bis 60 Gew.-% und besonders bevorzugt 30 bis 50 Gew-% mindestens eines Feuchthaltemittels,
- 25 bis 60 Gew.-%, bevorzugt 30 bis 55 Gew.-% und besonders bevorzugt 35 bis 50 Gew-% mindestens eines Putzkörpers und
- 0,05 bis 2 Gew.-%, bevorzugt 0,07 bis 0,5 Gew.-% und besonders bevorzugt 0,1 bis 0,2 Gew-% mindestens eines Tensids enthalten.

Wenn nicht anders angegeben beziehen sich alle Prozentangaben hierin auf die Gesamtmasse der Paste.

Es wurde überraschend gefunden, dass Prophylaxepasten mit der angegebenen Zusammensetzung auch unverpackt über längere Zeiträume aufbewahrt werden können, ohne dass sie ihre guten Verarbeitungseigenschaften verlieren. Sie behalten eine geschmeidige Konsistenz, d.h. sie trocknen und bluten nicht aus und werden nicht durch die Aufnahme von Umgebungsfeuchtigkeit verdünnt. Zudem ermöglichen sie eine schnelle und schonende Reinigung von Zahnoberflächen, ohne Restaurationsmaterialien zu zerkratzen, die weicher als natürlicher Zahnschmelz sind.

### Detaillierte Beschreibung der Erfindung

### Putzkörper

Die erfindungsgemäßen Polier- und Reinigungspasten enthalten 25 bis 60 Gew.-%, bevorzugt 30 bis 55 Gew.-% und besonders bevorzugt 35 bis 50 Gew-% mindestens eines Putzkörpers. Der oder die Putzkörper sind vorzugsweise aus Kaolin, Bimsmehl und Kieselsäure ausgewählt. Besonders bevorzugt sind Polier- und Reinigungspasten, die 0 bis 15 Gew.-% Kaolin, 0 bis 50 Gew.-% Bimsmehl und/oder 0 bis 30 Gew.-% Kieselsäure enthalten, wobei die Gesamtmenge der Putzkörper in den genannten Bereichen liegt.

Erfindungsgemäß sind solche Zusammensetzungen ganz besonders bevorzugt, die mindestens 10 Gew.-%, vorzugsweise 10 bis 50 Gew.-% und besonders bevorzugt 25 bis 35 Gew.-% Kaolin enthalten, bezogen auf die Gesamtmenge an Putzkörper.

Dentale Polier- und Reinigungspasten sollen einerseits effizient reinigen und andererseits die Zahnoberfläche so wenig wie möglich beschädigen. Bimsmehl zeichnet sich durch eine gute Reinigungswirkung aus, kann aufgrund seiner stark abrasiven Eigenschaften aber leicht zu einem Zerkratzen von Zahnoberflächen führen. Dies trifft insbesondere auf dentale Restaurationsmaterialien aus Kunststoff zu. Pyrogene und insbesondere gefällte Kieselsäuren haben demgegenüber eine amorphe Struktur und bestehen aus abgerundeten Partikeln, die kaum kratzen, dafür aber nur eine geringe Reinigungskraft haben und bei der professionellen Zahnreinigung Verfärbungen weniger schnell und effizient entfernen.

Erfindungsgemäß wurde überraschend gefunden, dass sich Kaolinpulver sehr gut zum Reinigen und Polieren von Zähnen eignet und eine effiziente und trotzdem schonende Reinigung von natürlichen und künstlichen Zahnoberflächen ermöglicht.

Erfindungsgemäß wird vorzugsweise möglichst reines Kaolin (CAS-Nr. 1332-58-7) verwendet, besonders bevorzugt Kaolin mit einem Kaolin-Gehalt von mehr als 90 Gew.-%, ganz besonders bevorzugt mit einem Kaolin-Gehalt von mehr als 98 Gew.-% und am meisten bevorzugt ca. 99 Gew.-%. Das Kaolin enthält vorzugsweise weniger als 1 Gew.-% kristallines Siliciumdioxid (Quarz). Gemäß einer besonders bevorzugten Ausführungsform wird Kaolin mit Pharmaqualität eingesetzt.

Bevorzugt ist weiterhin Kaolinpulver, das eine mittlere Partikelgröße (D50) von 1 bis 20 µm, besonders bevorzugt von 2 bis 15 µm und ganz besonders bevorzugt von 3 bis 10 µm aufweist. Darüber hinaus sind Kaolinpulver mit einem möglichst geringen Anteil grobkörniger Partikel bevorzugt. Der Anteil grobkörniger Partikel wird durch den D90-Wert charakterisiert. Kaolinpulver mit einer Partikelgröße (D90) von weniger als 30 µm, bevorzugt weniger als 20 µm und besonders bevorzugt weniger als 15 µm sind erfindungsgemäß besonders geeignet. Pulver, deren D50 und D90 Werte in den genannten Bereichen liegen, ermöglichen eine sehr schonende Reinigung von Zahnoberflächen ohne Restaurationsmaterialien zu zerkratzen.

Wenn nicht anders angegeben wird die Partikelgrößenverteilung von Pulvern mit einem Laserbeugungs-Partikelgrößenanalysator gemessen. Der D50-Wert ist kennzeichnend für die mittlere Partikelgröße in der Volumenverteilung und der D90 für den gröberen Anteil der Pulver. D50 bedeutet, dass die Hälfte des Pulvervolumens größer als der angegebene Wert ist, und D90, dass nur noch 10% des Pulvervolumens größer als der angegebene Wert sind.

Wenn nicht anders angegeben, handelt es sich bei allen Partikelgrößen um volumetrische Durchmesser, wobei die Partikelgrößenbestimmung im Bereich von 0,1 µm bis 1000 µm vorzugsweise mittels statischer Lichtstreuung erfolgt, beispielsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Die erfindungsgemäßen Prophylaxepasten enthalten bevorzugt Kaolinpulver mit einem D50-Wert von 1 bis 20 µm und einem D90-Wert unter 30 µm, bevorzugt einem D50-Wert von 2 bis 15 µm und einem D90-Wert unter 20 µm und besonders bevorzugt mit einem D50-Wert von 3 bis 10 µm und einem D90-Wert unter15 µm.

Die erfindungsgemäßen Polier- und Reinigungspasten können neben Kaolin zusätzlich weitere Putzkörper enthalten. Als weitere Putzkörper eignen sich besonders anorganische Pulver wie Calciumcarbonat, Calciumphosphat, Aluminiumoxid, Aluminiumoxyhydroxyhydrat [AlO(OH)·H₂O]. Besonders bevorzugt sind Bimssteinmehl sowie synthetische und natürliche Modifikationen von Siliciumdioxid. Eine bevorzugte, natürlich vorkommende Form von Siliciumdioxid ist Kieselgur. Bevorzugte Beispiele für synthetische Siliciumdioxidmodifikationen sind pyrogene und insbesondere gefällte Kieselsäuren. Besonders bevorzugt sind gefällte Kieselsäuren mit einer mittleren Partikelgröße (D50) von 5 bis 15 µm und einem D99-Wert < 30 µm und besonders bevorzugt < 25 µm.

Mischungen von Kaolinpulver und Kieselsäure sind erfindungsgemäß bevorzugt, ganz besonders Mischungen von Kaolinpulver und gefällter Kieselsäure (INCI-Bezeichnung: hydrated silca). Es wurde gefunden, dass mit Pasten, die eine Mischung von Kieselsäure und Kaolin enthalten, deutlich schneller eine gute Politur der Zähne erreicht werden kann, als mit Pasten, die ausschließlich amorphe Kieselsäuren enthalten. Trotzdem sind die Pasten auch gegenüber dentalen Kunststoffmaterialien ähnlich schonend. Ganz besonders bevorzugt sind Mischungen, die 20 bis 40 Gew.-%, besonders bevorzugt 24 bis 36 Gew.-% und am meisten bevorzugt 26 bis 34 Gew.-% Kaolin enthalten, bezogen auf die Gesamtmenge an Kaolin und Kieselsäure.

Durch Zugabe von Bimssteinmehl können Prophylaxepasten mit höherer Abrasivität hergestellt werden. Bimsstein ist ein poröses glasiges Vulkangestein. Chemisch gesehen ist es ein amorphes Aluminiumsilikat mit einer MOHS Härte von 6. Bimssteinmehl, kurz auch Bimsmehl genannt, ist ein fein gemahlenes Pulver aus Bimsstein. Da es vergleichbar hart wie Zahnschmelz ist, ist Bimsmehlpulver gut geeignet, um Beläge von natürlichen oder keramischen Zahnoberflächen zu entfernen. Erfindungsgemäß sind Bimssteinmehle mit einer mittleren Partikelgröße (D50) von 10 bis 60 µm, insbesondere 15 bis 50 µm und ganz besonders von 15 bis 40 µm. bevorzugt. Bei Bimssteinmehl ist es besonders wichtig, dass die gröbsten Partikel nicht zu groß sind, da diese die tiefsten Kratzer verursachen. Als Maß für die gröbsten Partikel kann der D99-Wert angenommen werden, d.h., dass 99% der Partikel sind kleiner als der genannte Wert. Bevorzugt ist ein Bimssteinmehl mit einem D99-Wert < 200 µm, besonders bevorzugt < 150 µm und ganz besonders bevorzugt < 120 µm.

Durch das Mischen unterschiedlicher Putzkörper kann die Abrasivität der Prophylaxepasten gezielt eingestellt werden. Bimsteinhaltige Prophylaxepasten eignen sich besonders zur Entfernung von Verfärbungen von den Zähnen. Bevorzugt sind Pasten, die Kieselsäure und Bimssteinmehl, eine Mischung von Kaolin, Kieselsäure und Bimssteinmehl oder eine Mischung von Kaolin und Kieselsäure enthalten. Pasten, die als Putzkörper ausschließlich Kaolin-Pulver und ggf. Kieselsäure enthalten, sind besonders bevorzugt, wobei in allen genannten Fällen gefällte Kieselsäure als Kieselsäure bevorzugt ist.

### Feuchthaltemittel

Die erfindungsgemäßen Polier- und Reinigungsmittel enthalten 25 bis 70 Gew.-%, bevorzugt 30 bis 60 Gew.-% und besonders bevorzugt 30 bis 50 Gew-% mindestens eines Feuchthaltemittels.

Erfindungsgemäß bevorzugte Feuchthaltemittel sind Glycerin, Sorbit, Xylit, Isomaltose, Erythrit, Propylencarbonat, Propylenglycol, Triethylenglyclol und Polyethylenglykol (PEG). Besonders bevorzugte Feuchthaltemittel sind Propylenglycol, Triethylenglycol, PEG 200 (CAS-Nr. 25322-68-3), insbesondere PEG 300 (CAS-Nr. 25322-68-3), PEG 400 (CAS-Nr. 25322-68-3) und ganz besonders bevorzugt Glycerin, Sorbitol und Xylit und Mischungen davon.

Bevorzugt sind Zusammensetzungen, die 18 bis 50 Gew.-%, besonders bevorzugt 25 bis 46 Gew.-% und ganz besonders bevorzugt 30 bis 44 Gew-% Glycerin enthalten.

Besonders bevorzugt sind Zusammensetzungen, die neben Glycerin zusätzlich 1 bis 5 Gew.-% Xylit und/oder 1 bis 5 Gew.-% Sorbit enthalten, wobei die Gesamtmenge an Xylit und Sorbitol vorzugsweise im Bereich von 1 bis 5 Gew.-% liegt.

Die Menge des oder der Feuchthaltemittel wird vorzugsweise so gewählt, dass das Gewichtsverhältnis von Wasser zur Gesamtmenge an Feuchthaltemittel(n) im Bereich von 0,05 bis 0,6, besonders bevorzugt von 0,25 bis 0,5 und ganz besonders bevorzugt im Bereich von 0,30 und 0,40 liegt.

### Tenside

Die erfindungsgemäßen Prophylaxepasten enthalten 0,05 bis 2 Gew.-%, bevorzugt 0,1 bis 0,5 Gew.-% und besonders bevorzugt 0,1 bis 0,2 Gew-% mindestens eines Tensids. Bevorzugte Tenside sind Natriumlaurylsulfat, Laurylglucosid und Natriumlauroylsacrosinat, wobei Natriumlaurylsulfat besonders bevorzugt ist.

### Verdickungsmittel

Zur Einstellung der Konsistenz enthalten die erfindungsgemäßen Prophylaxepasten vorzugsweise auch ein Verdickungsmittel.

Gemäß einer Ausführungsform enthalten die Pasten als Verdickungsmittel mindestens ein natürliches oder synthetisches Polymer mit verdickenden Eigenschaften. Bevorzugte Polymere sind Carboxymethylcellulose, Hydroxyethylcellulose, Methylcellulose, Guaran, Carrageen, Xanthan sowie Homo- oder Heteropolymere der Acrylsäure (Carbomere). Besonders bevorzugte Polymere sind Carbomere, Carboxymethylcellulose, Hydroxyethylcellulose und Xanthan. Carbomere werden vorzugsweise zusammen mit einem Neutralisierungsmittel eingesetzt. Bevorzugte Neutralisierungsmittel sind Natron- oder Kalilauge, Ammoniak, Arginin, Aminomethylpropanol, Tetrahydroxypropylethylendiamin, Ethylenedinitrilo)tetra-2-propanol, Triethanolamin, Tromethamin, PEG-15 Cocamine, Diisopropanolamin und Triisopropanolamin.

Die erfindungsgemäßen Prophylaxepasten weisen einen relativ geringen Wassergehalt auf. Viele polymere Verdickungsmittel sind jedoch wasserlöslich und erfordern daher einen hohen Wasseranteil. Carbomere, Xanthan und Hydroxyethylcellulose zeichnen sich dadurch aus, dass sie auch in wasserfreien oder wasserarmen Zusammensetzungen einsetzbar sind.

Polymeren Verdickungsmittel werden vorzugsweise in einer Menge von 0,2 bis 2 Gew.-%, besonders bevorzugt von 0,4 bis 1,5 Gew.-% und ganz besonders bevorzugt von 0,5 bis 1,0 Gew-% eingesetzt.

Gemäß einer bevorzugen Ausführungsform enthalten die erfindungsgemäßen Prophylaxepasten zur Verdickung mindestens eine Fettkomponente. Die Fettkomponente wird vorzugsweise in einer Menge von 0,5 bis 3 Gew.-%, besonders bevorzugt 0,7 bis 2,2 Gew.-% und ganz besonders bevorzugt 1 bis 1,3 Gew-% eingesetzt. Die Fettkomponente bewirkt durch Wechselwirkung mit dem oder den Tensiden, Wasser und Feuchthaltemittel eine Verdickung der Paste. Die Pasten können zusätzlich ein polymeres Verdickungsmittel enthalten, Pasten die keine polymeren Verdickungsmittel enthalten, sind jedoch bevorzugt.

Als Fettkomponente eignen sich besonders mit Wasser nicht mischbare Kohlenwasserstoffe und organische Verbindungen mit Kohlenwasserstoffresten, die jeweils Kohlenstoffketten mit mindestens 12 und ganz besonders bevorzugt mindestens 14 Kohlenstoffatomen enthalten. Bevorzugt sind Kohlenwasserstoffe und Kohlenwasserstoffreste, die Kohlenstoffketten mit 12 bis 30, besonders bevorzugt 12 bis 28, ganz besonders bevorzugt 14 bis 24 Kohlenstoffatomen und am meisten bevorzugt mit 16 bis 22 Kohlenstoffatomen enthalten. Bevorzugte Fettkomponenten sind Fettalkohole. Ganz besonders bevorzugt sind Fellalkohole mit einer Kettenlänge von 12 bis 22 und insbesondere 14 bis 22 Kohlenstoffatomen. Am meisten bevorzugt sind Fettalkohole mit 16 bis 18 Kohlenstoffatomen. Ganz besonders bevorzugte Fettalkohole sind Laurylalkohol und insbesondere Cetylalkohol und Stearylalkohol. Es können auch Mischungen verschiedener Fettkomponenten und insbesondere verschiedener Fettalkohole eingesetzt werden. Eine bevorzugte Mischung ist eine Mischung aus Cetyl- und Stearylalkohol, die im Folgenden auch als Cetylstearylalkohol bezeichnet wird, besonders bevorzugt eine ca. 1:1 Mischung.

Die Fettkomponente ist bei Raumtemperatur vorzugsweise fest und hat besonders bevorzugt einen Schmelzpunkt von ≥ 24°C, ganz besonders bevorzugt > 40°C. Am meisten bevorzugt sind Fettkomponenten mit einem Schmelzpunkt von 45 bis 60°C.

Die erfindungsgemäß bevorzugten Fettkomponenten zeichnen sich insbesondere dadurch aus, dass sie auch bei geringem Wassergehalt ausgezeichnete Verdickungseigenschaften haben.

### Metalloxide

Die erfindungsgemäßen Prophylaxepasten enthalten vorzugsweise mindestens ein Metalloxid. Das oder die Metalloxide sind vorzugsweise aus den Oxiden der Metalle der 4. Nebengruppe des Periodensystems der Elemente und den Oxiden vierwertiger Metalle (Me^{IV}O₂) ausgewählt, wobei Mischoxide vorzugsweise ausgeschlossen sind. Besonders bevorzugt sind Oxide des Zirkons und Titans.

Besonders bevorzugt sind die Oxide vierwertiger Metalle (Me^{IV}O₂), ganz besonders bevorzugt sind Zirkonoxid und insbesondere Titandioxid. Am meisten bevorzugt ist Anatas. Anatas ist eine der drei TiO₂-Modifikationen. Die beiden anderen Modifikationen sind Rutil und Brookit. Die Metalloxide sind vorzugsweise nicht oberflächenmodifiziert. Die genannten Metallverbindungen zeichnen sich durch eine außerordentlich polare Oberfläche aus.

Überraschenderweise wurde festgestellt, dass die Zugabe der genannten Metalloxide die Ausbildung eines Flüssigkeitsfilms auf der Oberfläche der Pasten, d.h. ein Ausbluten der Pasten, stark reduzieren oder sogar ganz verhindern kann, selbst bei der Lagerung bei 40°C und 75% rF. Die Pasten bleiben auch bei wechselnder Umgebungsluftfeuchtigkeit homogen. Dies gilt insbesondere für Pasten, die eine Fettkomponente als Verdickungsmittel enthalten. Die genaue Ursache für diesen Effekt ist nicht bekannt. Die Verwendung von Metalloxiden zum Verhindern des Ausblutens von dentalen Pasten ist ein Gegenstand der Erfindung.

Oxide von Metallen der 4. Nebengruppe werden vorzugsweise in einer Menge von 0,05 bis 3 Gew.-%, besonders bevorzugt 0,5 bis 3 Gew.-% und ganz besonders bevorzugt 0,5 bis 1,5 Gew.-% eingesetzt.

### Additive und Hilfsstoffe

Neben den genannten Komponenten können die erfindungsgemäßen Prophylaxepasten vorteilhaft einen oder mehrere Hilfsstoffe enthalten, wie beispielsweise Fluorid freisetzende Additive, Aromastoffe, Süßstoffe, Farbstoffe, Stabilisatoren und Mittel, die das Wachstum von Mikroorganismen verhindern.

Als Fluorid freisetzende Additive sind Fluoridverbindungen bevorzugt, insbesondere Natriumfluorid, Kaliumfluorid, Ammoniumfluorid, Ammoniumbifluorid, Natriummonofluorphosphat, Kaliummonofluorphosphat, Calciumfluorid, Salze von Tetra- oder Hexafluoranionen, wie z.B. Ammoniumhexafluorosilikat, Magnesiumhexafluorosilikat, Kaliumhexafluorphosphat, Ammoniumhexafluorotitanat, Ammoniumtetrafluorotitanat, Ammoniumhexafluoroaluminat. Zirkonfluorid, Tetra-n-butylammoniumdihydrogentrifluorid (TBAF-3), Rubidiumfluorid, Cäsiumfluorid, Kaliumbifluorid (KHF₂), Silber-I-fluorid (AgF), Zinn-II-fluorid (SnF₂), Olaflur und Dectaflur. Besonders bevorzugt sind: Natriumfluorid, Kaliumfluorid, Natriummonofluorphosphat, Zinn-II-fluorid (SnF₂), Ammoniumfluorid, Olaflur und Dectaflur.

Die Fluoridkomponente wird vorzugsweise in einer Konzentration von 0 bis 20.000 ppm, besonders bevorzugt von 100 bis 5000 ppm und ganz besonders bevorzugt von 500 bis 1500 ppm eingesetzt. Die Angaben beziehen sich auf die Menge des Fluoridanions in der Gesamtzusammensetzung.

Aromastoffe geben der Prophylaxepaste den gewünschten Geschmack. Bevorzugte Aromastoffe sind natürliche oder künstliche Zusammensetzungen mit Pfefferminz-, Erdbeer-, Himbeer-, Bananen-, Zitronen-, Kaugummi- oder Apfelgeschmack. Die Prophylaxepasten können einen oder mehrere Aromastoffe enthalten.

Die erfindungsgemäßen Prophylaxepasten können weiterhin einen oder mehrere Süßstoffe enthalten. Bevorzugte Süßstoffe sind Acesulfam, Aspartam, Cyclamat, Neohespiridin, Saccharin, Sucralose, Steviosid. Zuckeralkohole wie Xylit, Erythrit und Sorbit sind ebenfalls als Süßstoffe einsetzbar, erfordern jedoch relativ große Mengen.

### Wasseraktivität

Die erfindungsgemäßen dentalen Prophylaxepasten zeichnen sich dadurch aus, dass sie auch unter ungünstigen klimatischen Umgebungsbedingungen ihre geschmeidige Konsistenz beibehalten und selbst dann nicht austrocknen, wenn sie nicht dampfdicht verpackt sind. Dies wird dadurch erreicht, dass in den erfindungsgemäßen Pasten Art und Menge der verwendeten Komponenten in vorteilhafter Weise aufeinander abgestimmt sind. Es wurde gefunden, dass die Prophylaxepasten dann besonders gute Eigenschaften haben, wenn Art und Mengen der Komponenten so gewählt werden, dass die Pasten eine Wasseraktivität im Bereich von 0,5 bis 0,7, vorzugsweise 0,5 bis 0,65 und besonders bevorzugt von 0,55 bis 0,65 aufweisen, jeweils gemessen bei einer Temperatur von 25°C.

Die Wasseraktivität (aw) bei einer gegebenen Temperatur ist definiert als das Verhältnis des Wasserdampfpartialdrucks der Formulierung zum Sättigungsdampfdruck von reinem Wasser. Die Wasseraktivität der Pasten wird bestimmt, indem 3 g der Prophylaxepaste in eine hermetisch verschließbare Messkammer mit einem Volumen von 15 ml gegeben und anschließend die sich einstellende Luftfeuchtigkeit der eingeschlossenen Restluft gemessen wird. Die Luftfeuchtigkeit wird in % relative Luftfeuchtigkeit (rF) gemessen und steht mit dem aw-Wert in folgendem Zusammenhang: aw = rF/100. Die Messung erfolgt vorzugsweise mit einem handelsüblichen Messgerät, z.B. mit dem Modell LabTouch-aw advanced (Fa. Novasina AG, Neuheimstrasse 12, CH-8853 Lachen).

Die Einstellung der Wasseraktivität ermöglicht es, reproduzierbar Pasten mit den gewünschten Eigenschaften bereitzustellen. Die Wasseraktivität lässt sich besonders gut durch eine Variation der Mengen des oder der Feuchthaltemittel und des Wassers einstellen. Diese Mengen werden vorzugsweise so gewählt, dass der Wasseranteil im Bereich von 20 bis 30 Gew.-%, vorzugsweise 22 bis 29 Gew.-% und besonders bevorzugt 24 bis 28 Gew.-% liegt, bezogen auf die Gesamtmenge an Wasser und Feuchthaltemittel.

### Scheibenkonsistenz

Die erfindungsgemäßen Pasten zeichnen sich durch eine cremig geschmeidige Konsistenz aus, die eine gute Dosierbarkeit aus Tuben und eine gute Streichfähigkeit gewährleistet. Sie können daher gut mit dentalen Gummikelchen oder Polierbürstchen aufgenommen und auf den Zähnen verteilt werden. Gummikelche sind rotierende Dentalinstrumente, in der Form eines hohlen Zylinders. Sie werden zusammen mit einem rotierenden Handstück zur Reinigung oder Politur der Zähne eingesetzt. Typischerweise werden sie mit einem Andruck von ca. 2 N und einer Rotationsgeschwindigkeit von 2000 - 10.000 Umdrehungen pro Minute angewendet.

Als Maß für die Konsistenz der Pasten dient die Scheibenkonsistenz. Zur Messung der Scheibenkonsistenz wird eine definierte Menge (0,70 ± 0,01 g) der vortemperierten Probe (23 ± 2°C) zwischen zwei quadratischen Glasplatten (Dicke ca. 0,7 mm, Kantenlänge ca. 50 mm) mit einer definierten Prüflast (120 g) bei 23 ± 2°C für drei Minuten zusammengepresst. Anschließend wird der Durchmesser der zusammengepressten Probe ("Scheibendurchmesser") auf 0,01 mm genau ausgemessen. Der Durchmesser in mm entspricht der Scheibenkonsistenz.

Die erfindungsgemäßen Pasten haben vorzugsweise eine Scheibenkonsistenz von 20 bis 40 mm, besonders bevorzugt von 25 bis 38 mm und ganz besonders bevorzugt von 28 bis 35 mm.

### Fließeigenschaften

Die erfindungsgemäßen Prophylaxepasten zeichnen sich außerdem durch eine gute Standfestigkeit aus, so dass sie nicht aus Dosierhilfen, wie zum Beispiel Fingerclips herausfließen. Fingerclips sind kleine Gefäße mit ca. 2 ml Inhalt, welche mit einem Halter an den Fingern getragen werden können.

Zur Messung der Fließeigenschaften wird eine definierte Menge (1,5 ± 0,05 g) der vortemperierten Probe (23 ± 2°C) kreisförmig auf einem Mischblock (synthetisches Papier auf HDPE-Basis, z.B. Polyart^{®}, Fa. Arjobex, Boulogne-Billancourt, Frankreich) verteilt (Kreisdurchmesser ca. 2 cm). Anschließend wird der Mischblock mit der Probe für 15 ± 0,5 Minuten bei 23 ± 2°C waagrecht ruhen gelassen und dann senkrecht aufgestellt. Nach 15 ± 0,5 Minuten wird gemessen, um wie viele mm sich die Front der Paste nach unten bewegt hat. Die erfindungsgemäßen Pasten fliessen vorzugsweise weniger als 10 mm, besonders bevorzugt weniger als 3 mm und ganz besonders bevorzugt weniger als 1 mm.

Erfindungsgemäß sind Prophylaxepasten mit der folgenden Zusammensetzung besonders bevorzugt:
- 1 bis 20 Gew.-%, bevorzugt 1,5 bis 16 Gew.-% und besonders bevorzugt 12 bis 16 Gew-% Wasser,
- 13 bis 46 Gew.-%, bevorzugt 25 bis 46 Gew.-% und besonders bevorzugt 30 bis 40 Gew.-% Glycerin,
- 0 bis 25 Gew.-%, bevorzugt 1 bis 10 Gew.-% und besonders bevorzugt 1 bis 5 Gew-% Xylit und/oder Sorbit,
- 0 bis 45 Gew.-%, bevorzugt 10 bis 43 Gew.-% und besonders bevorzugt 12 bis 22 Gew-% Kaolin,
- 0 bis 40 Gew.-%, bevorzugt 0 bis 28 Gew.-% und besonders bevorzugt 20 bis 28 Gew-% Fällungskieselsäure,
- 0,05 bis 2 Gew.-%, bevorzugt 0,1 bis 0,5 Gew.-% und besonders bevorzugt 0,1 bis 0,2 Gew-% Natriumlaurylsulfat, und
- 0,5 bis 3 Gew.-%, bevorzugt 0,7 bis 2,0 Gew.-% und besonders bevorzugt 1 bis 1,3 Gew-% Cetylalkohol, Stearylalkohol oder eine Mischung davon,
   wobei die Gesamtmenge an Putzkörpern im Bereich von 36 bis 51 Gew.-% liegt.

Gemäß einer ganz besonders bevorzugten Ausführungsform enthalten die Pasten zusätzlich
- 0,5 bis 3,0, bevorzugt 0,7 bis 2,5, besonders bevorzugt 1,0 bis 2,0 Gew.-% Me^{IV}O₂, insbesondere TiO₂, ZrO₂ oder eine Kombinationen davon.

Weiter bevorzugt sind Prophylaxepasten mit der folgenden Zusammensetzung:
- 12 bis 16 Gew-% Wasser,
- 30 bis 40 Gew.-% Glycerin,
- 1 bis 2 Gew-% Xylit,
- 1 bis 3 Gew-% Sorbit,
- mindestens ein Putzkörper, ausgewählt aus 12 bis 22 Gew-% Kaolin, 20 bis 28 Gew-% Fällungskieselsäure, und 20 bis 50 Gew.-% Bimsmehl,
- 0,1 bis 0,2 Gew-% Natriumlaurylsulfat, und
- 1 bis 1,3 Gew-% Cetylalkohol, Stearylalkohol oder eine Mischung davon,
- 1,0 bis 2,0 Gew.-% TiO₂, ZrO₂ oder eine Kombinationen davon,
wobei die Gesamtmenge an Putzkörpern im Bereich von 36 bis 51 Gew.-% liegt.

In allen Fällen können die hierin genannten bevorzugten und besonders bevorzugten Bereiche unabhängig voneinander ausgewählt werden.

### Herstellungsverfahren

Die erfindungsgemäßen Prophylaxepasten lassen sich im einfachsten Fall herstellen, indem die einzelnen Komponenten homogen miteinander gemischt werden.

Pasten, die eine Fettkomponente als Verdickungsmittel enthalten, werden vorzugsweise hergestellt, indem man zunächst einen Teil des Wassers und des Feuchthaltemittels, vorzugsweise Wasser und Glycerin, vorgelegt und man dann die Putzkörper darin suspendiert. In diese Suspension können auch Farb-, Geschmackskomponenten und weitere optionale Additive wie z.B. die Fluoridkomponente eingearbeitet werden.

In einem separaten Behälter wird die Fettkomponente mit dem restlichen Anteil an Wasser und Feuchthaltemittel und dem Tensid gemischt und erhitzt, bis die Fettkomponente vollständig geschmolzen ist. Das Mischen wird fortgesetzt, bis eine homogene Mischung vorliegt.

Anschließend wird die Mischung der Fettkomponente zu der putzkörperhaltigen Suspension gegeben und bis zur Homogenität gemischt. Um den Einschluss von Luft zu verhindern erfolgt das Mischen vorzugsweise unter reduziertem Druck. Es wird eine cremig-geschmeidige, standfeste Paste erhalten.

Die einzelnen Bestandteile der erfindungsgemäßen Pasten werden vorzugsweise so auf die beiden Mischungen aufgeteilt, dass das Gewichtsverhältnis der putzkörperhaltigen Suspension zu der die Fettkomponente enthaltender Mischung im Bereich von 95:5 bis 50:50 liegt, wobei ein Verhältnis von 90:10 besonders bevorzugt ist.

Die erfindungsgemäßen Polier- und Reinigungspasten eignen sich besonders zur professionellen Zahnreinigung, d.h. zum professionellen Reinigen und Polieren der natürlichen Zähne, von Restaurationen und prothetischen Versorgungen. Für das schonende Reinigen von natürlichen Zähnen und insbesondere von Zähnen, die mit relativ weichen Füllungsmaterialien restauriert wurden, ist es wichtig, dass die Prophylaxepasten die Zahnoberflächen nicht zerkratzen. Herkömmliche Polierpasten mit hoher Reinigungsleistung weisen üblicherweise auch eine hohe Abrasivität auf, was ein hohes Verkratzungsrisiko mit sich bringt. Umgekehrt geht eine geringe Abrasivität typischerweise mit einer geringeren Reinigungsleistung einher, was den zeitlichen Aufwand zur Reinigung der Zähne erhöht. Derzeit werden die Zähne bei der professionellen Zahnreinigung in der Regel zunächst mit einer Paste mit hoher Reinigungswirkung behandelt und abschließend mit einer Paste mit geringer Abrasiviät poliert, um die Zahloberflächen zu glätten. Die erfindungsgemäßen Prophylaxepasten weisen eine für dentale Zwecke vorteilhafte Abrasivität und Reinigungswirkung auf, ohne Kratzer auf üblichen Kunststofffüllungsmaterialien und dentalen Metallen zu hinterlassen. Sie ermöglichen daher die Reinigung von Politur von Zahnoberflächen in nur einem Arbeitsgang.

Außerdem gewährleisten ihre Konsistenz und Standfestigkeit, dass sie nicht in kurzer Zeit von Reinigungsinstrumenten wie Bürstchen oder Gummikelchen wegfließen oder wegtropfen. Sie damit gut handhabbar und ermöglichen eine effiziente Reinigung sowohl von natürlichen Zahnoberflächen als auch von restaurierten Zahnoberflächen.

Die Pasten trocknen auch in trockener Umgebung selbst dann nicht aus, wenn sie offen oder in ungenügend dampfdichter Verpackung gelagert werden. In feuchter Umgebung bluten sie nicht aus und beginnen bei Feuchtigkeitsaufnahme nicht zu fließen. Sie bleiben cremig-geschmeidig und lassen sich in vielen Klimazonen einsetzen.

Die erfindungsgemäßen Prophylaxepasten können verpackt in Tuben oder Dosen vermarktet werden. Bevorzugt sind Single-Dose Verpackungen, die die für eine einmalige Anwendung erforderliche Menge an Prophylaxepaste enthalten, besonders bevorzugt ca. 1-2 ml. Als Verpackung werden vorzugsweise Kunststoffbehälter, insbesondere Tiefziehkunststoffbehälter, verwendet, die nach dem Befüllen mit einer Kunststoff-, Alu- oder Kunststoff/Alu-Laminatfolie verschlossen werden. Die Kunststoffbehälter werden vorzugsweise aus Polyethylen, Polyamid, Polypropylen, Polystyrol, Polyvinylchlorid oder Verbundmaterialien aus verschiedenen Kunststoffen hergestellt.

Im Folgenden wird die Erfindung anhand von Figuren und Ausführungsbespielen näher erläutert.
Figur 1 zeigt die Abrasivität (Reinigungsleistung) unterschiedlicher Prophylaxepasten. Die Paste R1-108 enthält ausschließlich Fällungskieselsäure und die Paste R3-27 ausschließlich Kaolin als Putzkörper. Die übrigen Pasten enthalten Mischungen von Fällungskieselsäure mit Kaolin unterschiedlicher Partikelgrößen. Es ist zu erkennen, dass der teilweise oder vollständige Ersatz der Fällungskieselsäure durch Kaolin die Reinigungsleistung der Pasten erhöht. Die Abrasivität nimmt mit zunehmender Partikelgröße des eingesetzten Kaolins zu.
Figur 2 zeigt die polierte Oberfläche eines handelsüblichen Kunststofffüllungsmaterials. Die Oberfläche ist glatt und weist keine sichtbaren Kratzer auf.
Figur 3 zeigt die das Kunststofffüllungsmaterial aus Figur 2 nach der Behandlung mit einer herkömmlichen Polierpaste. Die Paste hinterließ deutliche Kratzer auf der Oberfläche.
Figur 4 zeigt die das Kunststofffüllungsmaterial aus Figur 2 nach der Behandlung mit einer erfindungsgemäßen Polierpaste, die als Putzkörper eine Mischung aus Kaolin und Fällungskieselsäure enthält. Die Kunststoffoberfläche weist keine sichtbaren Kratzer auf.
Figur 5 zeigt eine rasterelektronenmikroskopische Aufnahme einer polierten Titan-Oberfläche. Die Oberfläche ist glatt und weist keine Kratzer auf.

Die Figuren 6 bis 9 zeigen rasterelektronenmikroskopische Aufnahmen von Titan-Oberflächen gemäß Figur 5, die mit herkömmlichen (Fig. 6 bis 8) bzw. einer erfindungsgemäßen Polierpaste behandelt wurden (Fig. 9). Die erfindungsgemäße Paste hinterließ keine Kratzer auf der Titanoberfläche, die mit herkömmlichen Pasten behandelten Oberflächen weisen deutlich sichtbare Kratzer auf.

Figur 10 zeigt herkömmliche und erfindungsgemäße Prophylaxepasten nach der Entnahme aus ihren Behältern. Das Produkt Proxyt RDA 7 fine und die erfindungsgemäßen Pasten R6-65 und R6-75 haben eine plastische, pastöse Konsistenz. Die übrigen Produkte haben eine krümelige Konsistenz und mussten mit einem Spatel aus den Einzeldosisverpackungen herausgenommen werden.

### Ausführungsbeispiele

### Beispiel 1:

### Herstellung und Untersuchung von Prophylaxepasten

Zur Herstellung der in Tabelle 1 beschriebenen Pasten wurden ein Großteil des Wassers und des Glycerins in einem Mischapparat vorgelegt und die Putzkörper darin suspendiert.

Anschließend wurden die Additive zugemischt. In einem separaten, beheizbaren Mischapparat wurden der Fettalkohol und das Tensid mit dem restlichen Anteil an Wasser und Feuchthaltemitteln gemischt und bis zum vollständigen Schmelzen des Fettalkohols erhitzt. Das Mischen wurde fortgesetzt, bis eine homogene Mischung vorlag. Dabei wurde der Fettalkohol durch das Tensid in der polaren Phase emulgiert. Anschließend wurde die Mischung des Fettalkohols zu der Suspension aus dem ersten Schritt gegeben und unter vermindertem Druck gemischt.

Alle Formulierungen in Tabelle 1 weisen eine Konsistenz und ein Fließverhalten auf, das für dentale Prophylaxepasten geeignet ist. Tabelle 1 zeigt, dass Pasten mit einer hohen Wasseraktivität schnell austrocknen und dann nicht mehr brauchbar sind (R3-88). Die Wasseraktivität, Scheibenkonsistenz und Fließeigenschaften wurden wie oben beschreiben gemessen. Bei geringer Wasseraktivität verhalten sich die Pasten hygroskopisch und nehmen aus der Luftfeuchtigkeit Wasser auf, was deren Verarbeitungseigenschaften verschlechtert (R1-108, R3-89). Bei den Pasten R1-108 und R3-89 führte die Wasseraufnahme dazu, dass sich auf der Oberfläche des Materials ein flüssiger Film bildete, was die Verarbeitungseigenschaften des Materials beeinträchtigt. Die Pasten R3-52, R3-53, R3-27 bluten nur wenig aus, und bei den Pasten N1-152, R6-65, R6-75, R3-54, R3-80 und R6-73 ist das Ausbluten nochmals weiter verringert.

Ein Vergleich der Pasten R3-89 und R3-54 zeigt, dass ein Ausbluten dann besonders wirksam verhindert wird, wenn die Wasseraktivität über 0,5 liegt und die Paste ein Metalloxid enthält.

Die Wasseraufnahme und der Wasserverlust der Pasten wurden unter extremen Umgebungsbedingungen bestimmt. Hierzu wurde quantifiziert, wie viel Gewicht die Pasten bei Trockenlagerung bei Raumtemperatur 23±2°C (RT) bei ca. 10% relative Luftfeuchtigkeit (rF)) oder unter tropischen Bedingungen (40°C, 75% rF) aufnahmen oder verloren. Die Trockenlagerung wurde in einem Exsikkator über Lithiumchlorid durchgeführt. In den Exsikkator wurde ein digitaler Feuchtigkeitsmesser gelegt, so dass das Austrocknen verfolgt werden konnte. Dabei wurden die Proben so lange im Exsikkator gelagert, bis sich eine Gleichgewichtsfeuchte von ca. 10% eingestellt hatte. Dies war typischerweise nach 10 bis 14 Tagen der Fall. Die Lagerung unter tropischen Bedingungen erfolgte in einem Klimaschrank mit Temperatur- und Feuchteregelung. Die Proben wurden ca. 2 Wochen unter diesen Bedingungen gelagert und hatten in dieser Zeit das Feuchtegleichgewicht mit der Umgebung erreicht. In der Tabelle 2 sind beispielhaft die gemessenen Werte für zwei Pasten gezeigt.

**Tabelle 1: Zusammensetzung und Eigenschaften der Pasten**

| **Bestandteil** | | **Beispiel [Gew,-%]** | | | | | | | | | | | |
|---|---|---|---|---|---|---|---|---|---|---|---|---|---|
| | | **R1-108*)** | **N1-152** | **R3-89*)** | **R3-88*)** | **R6-65** | **R6-75** | **R3-54** | **R3-52** | **R3-80** | **R3-53** | **R3-27** | **R6-73** |
| **Lösungsmittel** | Wasser | 12,20 | 14,20 | 0,23 | 57,31 | 15,98 | 12,91 | 14,20 | 14,20 | 14,20 | 14,20 | 14,20 | 14,90 |
| **Feuchthaltemittel** | Glycerin | 45,22 | 43,09 | 57,08 | 0 | 39,09 | 31,28 | 43,11 | 43,11 | 43,09 | 43,11 | 43,11 | 36,19 |
| | Xylitol | 1 | 1 | 1 | 1 | 1 | 1 | 1 | 1 | 1 | 1 | 1 | 1 |
| | Sorbitol | 0 | 0 | 0 | 0 | 2,1 | 1,75 | 0 | 0 | 0 | 0 | 0 | 2,1 |
| **Tensid** | Na-Laurylsulfat¹⁾ | 0,12 | 0,12 | 0,12 | 0,12 | 0,14 | 0,12 | 0,12 | 0,12 | 0,12 | 0,12 | 0,12 | 0,2 |
| **Fettkomponente** | Cetylstearylalkohol²⁾ | 1,06 | 1,06 | 1,06 | 1,06 | 1,27 | 1,06 | 1,06 | 1,06 | 1,06 | 1,06 | 1,06 | 1,27 |
| **Metalloxid** | TiO₂¹⁵⁾ | 0,05 | 1 | 1 | 1 | 1 | 2 | 1 | 0 | 0 | 0 | 0,15 | 1 |
| | ZrO₂ | 0 | 0 | 0 | 0 | 0 | 0 | 0 | 0 | 2 | 0 | 0 | 0 |
| **Putzkörper** | Kaolin³⁾ | 0 | 12⁵⁾ | 12⁶⁾ | 12⁶⁾ | 12⁵⁾ | 0 | 12⁶⁾ | 12⁶⁾ | 12⁵⁾ | 12⁵⁾ | 40⁷⁾ | 0 |
| | Kieselsäure⁴⁾ | 40 | 27,15 | 27,15 | 27,15 | 27 | 0 | 27,15 | 28,15 | 26,15 | 28,15 | 0 | 23 |
| | Bimssteinmehl | 0 | 0 | 0 | 0 | 0 | 49,48 | 0 | 0 | 0 | 0 | 0 | 20 |
| **Additive** | NaF | 0,12 | 0,12 | 0,12 | 0,12 | 0,12 | 0,12 | 0,12 | 0,12 | 0,12 | 0,12 | 0,12 | 0,12 |
| | Antischaummittel | 0,1 | 0,1 | 0,1 | 0,1 | 0,1 | 0,1 | 0,1 | 0,1 | 0,1 | 0,1 | 0,1 | 0,1 |
| | Pigmente | 0,01 | 0,04 | 0,02 | 0,02 | 0,08 | 0,06 | 0,02 | 0,02 | 0,04 | 0,02 | 0,02 | 0,08 |
| | Aroma | 0,12 | 0,12 | 0,12 | 0,12 | 0,12 | 0,12 | 0,12 | 0,12 | 0,12 | 0,12 | 0,12 | 0,1 |
| **Summe** | | **100** | **100** | **100** | **100** | **100** | **100** | **100** | **100** | **100** | **100** | **100** | **100** |
| **Eigenschaften** | Konsistenz [mm]⁸⁾ | 32 | 33 | 33 | 50 | 30 | 29 | 33 | 31 | 31 | 33 | 38 | 31 |
| | Fließen [mm] | 0,0 | 0,0 | 6,1 | > 20 | 0,0 | 0,0 | 0,5 | 0,0 | 0,56 | 0,0 | 0,0 | 0,0 |
| | Wasseraktivität | 0,48 | 0,56 | 0,13 | 0,97 | 0,60 | 0,6 | 0,56 | 0,56 | 0,56 | _16) | 0,51 | 0,60 |
| | Brauchbarkeit nach Trocknen⁹⁾ | ja | ja | ja | nein | ja | ja | ja | ₋¹⁶⁾ | -¹⁶⁾ | ja | ja | ja |
| | Ausbluten bei 40°C, 75% ^{10),14)} | +++ | + | +++ | 0¹²⁾ | + | 0 | 0 | ++ | + | ++ | ++ | 0 |
| | Ausbluten bei 25°C, 60% ^{11),14)} | +++ | 0 | +¹³⁾ | 0¹²⁾ | 0 | 0 | -¹⁶⁾ | -¹⁶⁾ | -¹⁶⁾ | -¹⁶⁾ | -¹⁶⁾ | 0 |

| | | | | | | | | | | | | | |
|---|---|---|---|---|---|---|---|---|---|---|---|---|---|
| *) Vergleichsbeispiel ¹⁾ CAS-Nr. 151-21-3 ²⁾ CAS-Nr. 67762-27-0 ³⁾ CAS-Nr. 1332-58-7 ⁴⁾ Fällungskieselsäure, mittlere Partikelgröße (D50) 5,5 - 9,5 µm, CAS-Nr. 112926-00-8 ⁵⁾ D50 ∼3,9 µm ⁶⁾ D50 ∼ 6,6 µm ⁷⁾ D50 ∼ 8,0 µm ⁸⁾ Scheibenkonsistenz ⁹⁾ Lagerung für 2 Wochen bei 23±2 °C und 10% rF (Exsikkator über Lithiumchlorid) ¹⁰⁾ bei Lagerung für 2 Wochen bei 40°C und 75% rF (Klimaschrank mit Temperatur- und Feuchteregelung); ¹¹⁾ bei Lagerung für 2 Wochen bei 25°C und 60% rF (Klimaschrank mit Temperatur- und Feuchteregelung); ¹²⁾ Paste trocknete aus ¹³⁾ Paste wurde dünnflüssig ¹⁴⁾ +++ = Paste blutet stark; ++ = wenig; + = sehr wenig, 0 = nicht aus ¹⁵⁾ Anatas; CAS-Nr. 13463-67-7 ¹⁶⁾ nicht gemessen | | | | | | | | | | | | | |

Die in Tabelle 2 genannten Prophylaxepasten weisen eine Wasseraktivität von 0,56 auf und bluten selbst bei einer Luftfeuchtigkeit von 75% rF kaum aus. Die gefundenen Unterschiede in der Gewichtsveränderung sind vernachlässigbar. Beide Pasten nehmen in etwa gleich viel Feuchtigkeit aus der Umgebung auf und geben gleich viel Feuchtigkeit ab. Die Paste R3-54 zeichnet sich dadurch aus, dass sie im Vergleich zu der Paste R3-52 überhaupt nicht ausblutet. Dies ist auf den Gehalt an Titandioxid zurückzuführen. Die Pasten wurden bei der Lagerung bei Raumtemperatur (10% rF) zwar fester, konnten aber immer noch gut mit dentalen Gummikelchen aufgenommen werden und waren uneingeschränkt brauchbar. Auch nach Lagerung bei 40°C (75% rF) blieben die Pasten geschmeidig, konnten gut in Gummikelche aufgenommen werden und waren ohne Einschränkung brauchbar.

**Tabelle 2: Gewichtsveränderungen der Pasten aus Tabelle 1 bei offener Lagerung unter verschiedenen Umgebungsbedingungen**

| **Charge** | **R3-52** | **R3-54** |
|---|---|---|
| Gewichtsverlust bei RT, ca. 10% rF | 11,4 Gew% | 11,3 Gew% |
| Gewichtszunahme bei 40°C, 75% rF | 14,5 Gew% | 14,3 Gew% |

### Beispiel 2:

### Messung der Abrasivität von Prophylaxepasten

Zur Bestimmung der Abrasivität (Reinigungsleistung) der Pasten wurden Zylinder aus reinem Kupfer (Durchmesser von 25 mm) mit den Pasten poliert und der Materialabtrag gemessen. Reines Kupfer ist weicher als dentale Kunststofffüllungsmaterialen und übliche Dentallegierungen. Das Verfahren erlaubt im Labor eine Messung der Abrasivität unter reproduzierbaren Bedingungen. Es wurde eine handelsübliche Schleif- und Poliermaschine eingesetzt (Buehler EcoMet250 mit AutoMet250 Schleifkopf). Ein Poliertuch mit einem Durchmesser von 10 inch (25,4 cm) (Typ TexMet C; Buehler, Lake Bluff, IL, USA Art. 40-1110) wurde mit Hilfe eines Spatels mit einer dünnen Schicht (ca. 15 g) der zu untersuchenden Prophylaxepaste bestrichen. Anschließend wurden die Kupferzylinder mit der Schleifmaschine ohne Wasserkühlung für 5 Minuten unter folgenden Bedingungen poliert:

| | |
|---|---|
| • Geschwindigkeit Kopf: | 30 rpm |
| • Geschwindigkeit Basis: | 100 rpm |
| • Einzelanpressdruck: | 20 N |
| • Gleich-/Gegenlauf: | Gleichlauf |

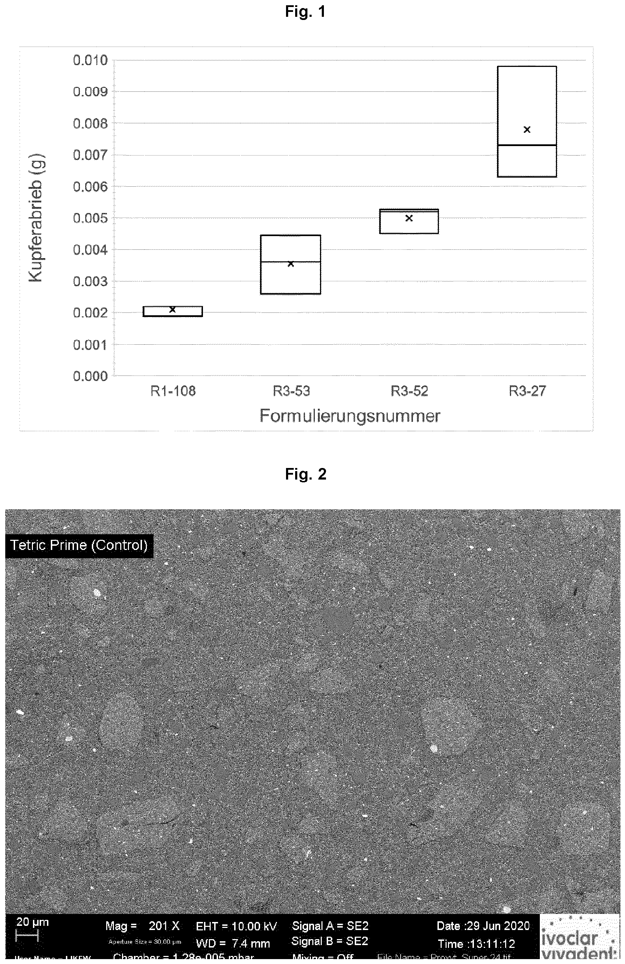

Es wurden Prophylaxepasten ohne (Paste R1-108*) und mit Kaolin (Pasten R3-53, R3-52 und R3-27) verglichen. Die Pasten enthielten Kaolin mit unterschiedlichen Partikelgrößen. Die Abrasion an den Kupferprüfkörpern wurde quantifiziert, indem die Gewichtsdifferenz vor und nach dem Polieren mit einer Analysenwaage ermittelt wurde. Figur 1 zeigt die Abrasivität der untersuchten Prophylaxepasten. Es ist zu erkennen, dass der teilweise oder vollständige Ersatz der Fällungskieselsäure durch Kaolin die Abrasivität und damit die Reinigungsleistung von Prophylaxepasten erhöht. Die Abrasivität nimmt mit zunehmender mittlerer Partikelgröße des eingesetzten Kaolins zu.

### Beispiel 3:

### Bestimmung der Kratzeigenschaften von Prophylaxepasten auf Kunststofffüllungsmaterialien

Das Kratzverhalten einer erfindungsgemäßen Polierpaste wurde mit dem eines marktüblichen Produkts verglichen. Hierzu wurde ein Kompositfüllungsmaterial (Tetric Prime der Farbe A3; Ivoclar Vivadent AG, Schaan, Liechtenstein) gemäß der Gebrauchsanweisung des Herstellers polymerisiert, anschließend geschliffen und dann unter Wasserkühlung mit 4000 grit Siliciumcarbidpapier poliert. Figur 2 zeigt die polierte Oberfläche des Kunststofffüllungsmaterials. Es ist eine glatte Oberfläche ohne Kratzer sichtbar.

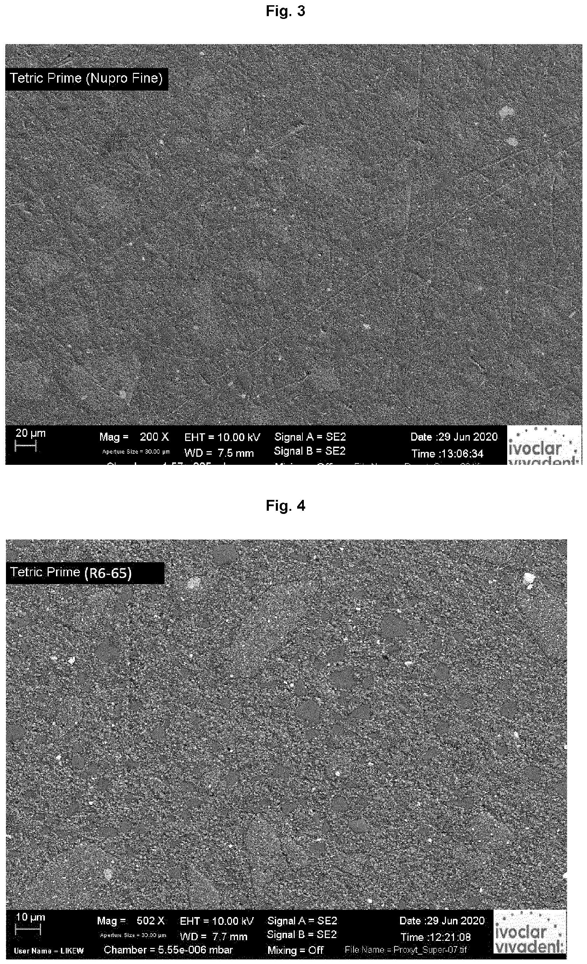

Die glatte Oberfläche wurde anschließend mit einer handelsüblichen Polierpaste (Nupro Fine, Dentsply Professional, York PA, USA, Artikelnummer 801232, Lot: 00022171) für 10 s und 2 N Anpressdruck bei 3000 Umdrehungen pro Minute mit einem dentalen Gummikelch poliert. Das Produkt hinterließ deutliche Kratzer auf der Oberfläche (Figur 3).

Zum Vergleich wurde die glatte Oberfläche des Kompositfüllungsmaterials mit der erfindungsgemäßen Paste R6-65 auf die beschriebene Weise behandelt. Figur 4 zeigt, dass die erfindungsgemäße Polierpaste, die als Putzkörper eine Mischung aus einer Fällungskieselsäure und Kaolin enthält, nicht zu einer Verkratzung der Kunststoffoberfläche führte.

Die Ergebnisse der Beispiel 2 und Beispiel 3 zeigen, dass erfindungsgemäße Prophylaxepasten eine für dentale Zwecke vorteilhafte Abrasivität und Reinigungswirkung aufweisen, ohne übliche Kunststofffüllungsmaterialien zu zerkratzen. Die kaolinhaltigen Pasten waren vergleichbar schonend wie Fällungskieselsäure, erzielten aber eine deutlich bessere Reinigungswirkung, so dass der Reinigungsvorgang deutlich weniger Zeit in Anspruch nimmt.

### Beispiel 4:

### Kratzeiaenschaften von Prophylaxepasten auf dentalem Titan

Titan Grad 5 wir zur Herstellung von Implantataufbauten eingesetzt. Auch diese Teile können in der Mundhöhle aufgesetzt sein und müssen gereinigt werden. Da viele Metalle und insbesondere Titan Grad 5 noch kratzempfindlicher als dentale Kunststofffüllungsmaterialen sind, ist hier ein besonders schonendes Vorgehen erforderlich, um die Oberfläche nicht zu beschädigen.

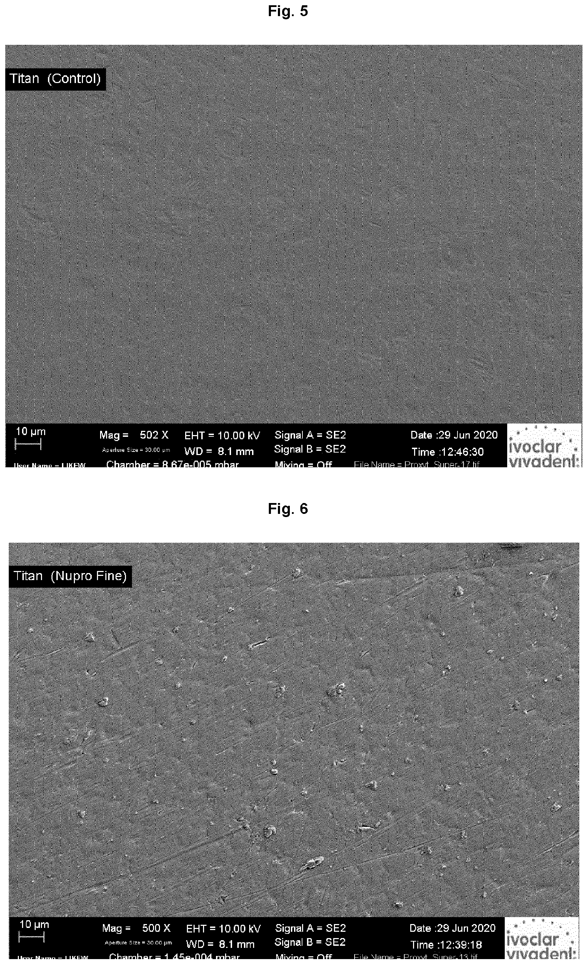

Zum Vergleich der Kratzeigenschaften erfindungsgemäßer Polierpasten und handelsüblicher Materialien wurde Titan Grad V poliert. Hierzu wurden runde Titanplättchen (19 mm Durchmesser, 2,25 mm Dicke) in GTS Polystyrolgießharz eingebettet. Danach wurden die Plättchen mit 4000 grit Siliciumcarbidpapier nass geschliffen und anschließend mit einem Poliertuch (Chemomet Polishing Cloth Art. 40-7920, Buehler, LakeBuff, IL, USA) und einer Sequenz von Poliermitteln (1. Buehler PasterPrep Polishing Suspension 0,05µm, Art. 40-6377-032; 2. Buehler MasterMet Colloidal Silica Suspension, Art. 40-6370-006; 3. Buehler MasterMet 2 Non-crystallizing colloidal silica polishing suspension, Art. 40-6380-006) auf Hochglanz poliert. In jedem Schritt wurden die Plättchen für 10 min bei 100 rpm und einem Anpressdruck von 20 N bzw. im letzten Schritt nur 15 N poliert. Figur 5 zeigt eine rasterelektronenmikroskopische Aufnahme der polierten Oberfläche. Die Oberfläche ist glatt und weist keine Kratzer auf.

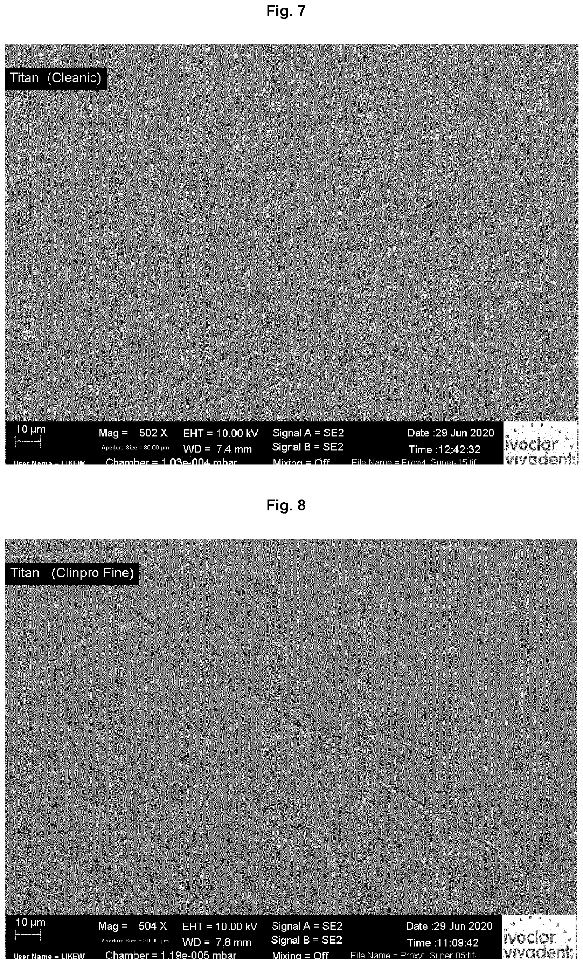

Anschließend wurde die Titanoberfläche für 10s und 2 N Anpressdruck mit einem dentalen Gummikelch bei 3000 Umdrehungen pro Minute mit den zu untersuchenden Pasten poliert. Es wurden die folgenden Materialien verwendet:
- Nupro Fine (Dentsply Professional, York PA, USA, Artikelnummer 801232, Lot: 00022171; Figur 6)
- Cleanic (KerrHawe SA, Bioggo, Switzerland, Artikelnummer 3140, Lot: 7251052; Figur 7)
- Clinpro (3M Oral Care Solutions Division, St. Paul MN, USA. Artikelnummer 12613, Lot: 091119A; Figur 8)

Die Figuren 6 bis 8 zeigen die Oberfläche des Titans nach der Behandlung. Die Produkte haben in allen Fällen deutliche Kratzer auf der Oberfläche hinterlassen.

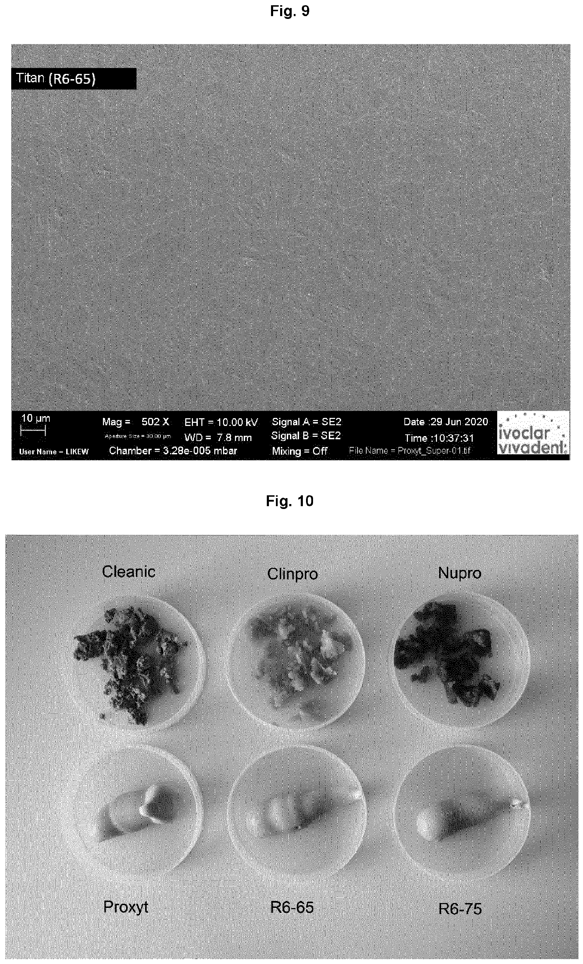

Zum Vergleich wurde die Titan-Oberfläche mit der erfindungsgemäßen Paste R6-65 auf die beschriebene Weise behandelt. Figur 9 zeigt, dass die erfindungsgemäße Polierpaste keinerlei Kratzer auf dem Titan hinterlassen hat.

### Beispiel 5:

### Vergleich der Konsistenz von erfindungsgemäßen Pasten und handelsüblichen Produkten

Die meisten handelsüblichen Prophylaxepasten werden in kleinen Einzelverpackungen vertrieben und enthalten nur wenig Wasser. Sie haben eine relativ harte Konsistenz. Beispiele hierfür sind die Produkte Nupro (Dentsply Professional, York PA, USA), Clinpro (3M Oral Care Solutions Division, St. Paul MN, USA) und Cleanic (KerrHawe SA, Bioggo, Switzerland). Das Produkt Proxyt RDA 7 fine (Ivoclar Vivadent AG, Schaan, Liechtenstein) wird in Tuben angeboten.

Figur 10 zeigt die oben genannten Produkte nach der Entnahme aus ihren Behältern. Es ist sichtbar, dass nur Proxyt RDA 7 fine eine plastische pastöse Masse ist. Die anderen Produkte mussten mit einem Spatel aus den Einzeldosisverpackungen herausgenommen werden. Sie sind schwer mit Gummikelchen aufzunehmen.

Zum Vergleich werden auch die erfindungsgemäßen Prophylaxepasten R6-65 (Lot: YM1278) und R6-75 gezeigt. Die Pasten R6-65 und R6-75 haben wie das Produkt Proxyt RDA 7 fine eine cremig-geschmeidige Konsistenz und können sowohl in Tuben wie auch in kleinen Single-Dose Verpackungen gelagert werden. Aus einer Tube können sie leicht dosiert werden.

Die Pasten wurden unter verschiedenen Bedingungen gelagert (vgl. Tabelle 3). Die erfindungsgemäße Paste R6-65 änderte ihre Eigenschaften dabei kaum. Sie blieb über einen großen Luftfeuchtigkeitsbereich (von 10% rF bis 75% rF) homogen und plastisch und kann daher problemlos auch in Einzelverpackungen aufbewahrt werden. Sie bleibt selbst in der geöffneten Verpackung über einen längeren Zeitraum cremig-geschmeidig.

Das Produkt Proxyt RDA 7 fine war anfangs ebenfalls pastös, trocknete jedoch relativ schnell aus. Die übrigen Pasten behielten ihre krümelige Konsistenz bei und waren vergleichsweise schwer zu verarbeiten.

**Tabelle 3: Eigenschaften von erfindungsgemäßen Prophylaxepasten im Vergleich zu Marktprodukten**

| **Eigenschaft** | **R6-65** | **R6-75** | **Proxyt RDA 7 fine¹⁾** | **Nupro²⁾** | **Clinpro³⁾** | **Cleanic⁴⁾** |
|---|---|---|---|---|---|---|
| Scheiben konsistenz (mm) | 30 | 29 | 32 | Nicht messbar | Nicht messbar | Nicht messbar |
| Fließen (mm) | 0,0 | 0,0 | 4,0 | Nicht messbar | Nicht messbar | Nicht messbar |
| Konsistenz in Verpackung | crèmiggeschmeidig | crèmiggeschmeidig | Pastös | Fest | Fest | Fest |
| Verhalten beim Dosieren | weich plastisch | weich plastisch | zähplastisch | krümelig | krümelig | krümelig |
| Offenlagerung bei 23°C, 10% rF | geschmeidig | geschmeidig | ausgetrocknet | --⁵⁾ | --⁵⁾ | --⁵⁾ |
| Offenlagerung bei 25°C, 60% rF | crèmiggeschmeidig | crèmiggeschmeidig | ausgetrocknet | --⁵⁾ | --⁵⁾ | --⁵⁾ |
| Offenlagerung bei 40°C, 75% rF | crèmiggeschmeidig | crèmiggeschmeidig | pastös | --⁵⁾ | --⁵⁾ | --⁵⁾ |

| | | | | | | |
|---|---|---|---|---|---|---|
| ¹⁾ Proxyt RDA 7 - fein (Ivoclar Vivadent AG, Schaan, Liechtenstsein, Artikelnummer 579866, Lot: Y51058) ²⁾ Nupro (Dentsply Professional, York PA, USA, Artikelnummer 801232, Lot: 00022171) ³⁾ Clinpro (3M Oral Care Solutions Division, St. Paul MN, USA. Artikelnummer 12613, Lot: 091119A) ⁴⁾ Cleanic (KerrHawe SA, Bioggo, Switzerland, Artikelnummer 3140, Lot: 7251052) ⁵⁾ nicht gemessen | | | | | | |

## Patentansprüche

1. Dentale Polier- und Reinigungspaste, die
1 bis 30 Gew.-% Wasser,
25 bis 70 Gew.-% mindestens eines Feuchthaltemittels,
25 bis 60 Gew.-% mindestens eines Putzkörpers und
0,05 bis 2 Gew.-% mindestens eines Tensids enthält,
jeweils bezogen auf die Gesamtmasse der Polier- und Reinigungspaste.

2. Polier- und Reinigungspaste nach Anspruch 1, die als Putzkörper 0 bis 15 Gew.-% Kaolin und/oder 0 bis 50 Gew.-% Bimsmehl und/oder 0 bis 30 Gew.-% Kieselsäure enthält, jeweils bezogen auf die Gesamtmasse der Polier- und Reinigungspaste, wobei die Gesamtmenge der Putzkörper in dem in Anspruch 1 genannten Bereich liegt.

3. Polier- und Reinigungspaste nach Anspruch 1 oder 2, in der das Kaolinpulver einer mittlere Partikelgröße (D50) von 1 bis 20 µm, bevorzugt von 2 bis 15 µm und ganz besonders bevorzugt von 3 bis 10 µm und/oder eine Partikelgröße (D90) von weniger als 30 µm, bevorzugt weniger als 20 µm und besonders bevorzugt weniger als 15 µm aufweist.

4. Polier- und Reinigungspaste nach einem der vorhergehenden Ansprüche, die mindestens 10 Gew.-%, vorzugsweise 10 bis 50 Gew.-% und besonders bevorzugt 25 bis 35 Gew.-% Kaolin enthält, bezogen auf die Gesamtmenge an Putzkörper.

5. Polier- und Reinigungspaste nach einem der vorhergehenden Ansprüche, die als Putzkörper eine Mischung von Kieselsäure und Kaolinpulver und vorzugsweise keine weiteren Putzkörper enthält.

6. Polier- und Reinigungspaste nach einem der vorhergehenden Ansprüche, die zusätzlich 0,5 bis 3 Gew.-%, bevorzugt 0,7 bis 2,2 Gew.-% und ganz besonders bevorzugt 1 bis 1,3 Gew-% mindestens einer Fettkomponente enthält, bezogen auf die Gesamtmasse der Polier- und Reinigungspaste.

7. Polier- und Reinigungspaste nach Anspruch 6, die als Fettkomponente einen Fellalkohol mit einer Kettenlänge von 12 bis 22, vorzugsweise 14 bis 22 und besonders bevorzugt mit 16 bis 18 Kohlenstoffatomen enthält.

8. Polier- und Reinigungspaste nach einem der vorhergehenden Ansprüche, die zusätzlich 0,05 bis 3 Gew.-%, bevorzugt 0,5 bis 3 Gew.-% und ganz besonders bevorzugt 0,5 bis 1,5 Gew.-% mindestens eines Metalloxids enthält, bezogen auf die Gesamtmasse der Polier- und Reinigungspaste, vorzugsweise Titandioxid und/oder Zirkonoxid, besonders bevorzugt Anatas.

9. Polier- und Reinigungspaste nach einem der vorhergehenden Ansprüche, die zusätzlich mindestens ein Additiv enthält, das aus Fluoridquellen, Süßstoffen, Aromastoffen und Mischungen davon aus gewählt ist.

10. Polier- und Reinigungspaste nach einem der vorhergehenden Ansprüche, die eine Wasseraktivität von 0,5 bis 0,70, vorzugsweise 0,5 bis 0,65 und besonders bevorzugt von 0,55 bis 0,65 aufweist, gemessen bei einer Temperatur von 25°C.

11. Polier- und Reinigungspaste nach einem der vorhergehenden Ansprüche, die eine Scheibenkonsistenz von 20 bis 40 mm, besonders bevorzugt von 25 bis 38 mm und ganz besonders bevorzugt von 28 bis 35 mm aufweist.

12. Polier- und Reinigungspaste nach einem der vorhergehenden Ansprüche, die weniger als 10 mm, bevorzugt weniger als 3 mm und ganz besonders bevorzugt weniger als 1 mm fließt, wenn 1,5 ± 0,05 g der vortemperierten Probe (23 ± 2°C) kreisförmig auf einem Mischblock verteilt (Kreisdurchmesser ca. 2 cm) werden, der Mischblock anschließend mit der Probe für 15 ± 0,5 Minuten bei 23 ± 2°C waagrecht ruhen gelassen und dann senkrecht aufgestellt wird und dann nach 15 ± 0,5 Minuten gemessen wird, um wie viele mm sich die Front der Paste nach unten bewegt hat.

13. Polier- und Reinigungspaste nach einem der vorhergehenden Ansprüche, bei dem die Menge des oder der Feuchthaltemittel so gewählt ist, dass das Gewichtsverhältnis von Wasser zur Gesamtmenge an Feuchthaltemittel(n) im Bereich von 0,05 bis 0,6, besonders bevorzugt von 0,25 bis 0,5 und ganz besonders bevorzugt im Bereich von 0,30 und 0,40 liegt.

14. Dentale Polier- und Reinigungspaste nach einem der vorhergehenden Ansprüche die
- 1 bis 20 Gew.-%, bevorzugt 1,5 bis 16 Gew.-% und besonders bevorzugt 12 bis 16 Gew-% Wasser,
- 13 bis 46 Gew.-%, bevorzugt 25 bis 46 Gew.-% und besonders bevorzugt 30 bis 40 Gew.-% Glycerin,
- 0 bis 25 Gew.-%, bevorzugt 1 bis 10 Gew.-% und besonders bevorzugt 1 bis 5 Gew-% Xylit und/oder Sorbit,
- 0 bis 45 Gew.-%, bevorzugt 10 bis 43 Gew.-% und besonders bevorzugt 12 bis 22 Gew-% Kaolin,
- 0 bis 40 Gew.-%, bevorzugt 0 bis 28 Gew.-% und besonders bevorzugt 20 bis 28 Gew-% Fällungskieselsäure,
- 0,05 bis 2 Gew.-%, bevorzugt 0,1 bis 0,5 Gew.-% und besonders bevorzugt 0,1 bis 0,2 Gew-% Natriumlaurylsulfat,
- 0,5 bis 3 Gew.-%, bevorzugt 0,7 bis 2,0 Gew.-% und besonders bevorzugt 1 bis 1,3 Gew-% Cetylalkohol, Stearylalkohol oder eine Mischung davon, und
- ggf. 0,5 bis 3,0, bevorzugt 0,7 bis 2,5, besonders bevorzugt 1,0 bis 2,0 Gew.-% Me^{IV}O₂, insbesondere TiO₂, ZrO₂ oder eine Kombinationen davon, enthält,
jeweils bezogen auf die Gesamtmasse der Polier- und Reinigungspaste, wobei die Gesamtmenge an Putzkörpern im Bereich von 36 bis 51 Gew.-% liegt.

15. Verwendung einer Polier- und Reinigungspaste gemäß einem der vorhergehenden Ansprüche zur Reinigung und Politur von Zähnen.
